(19) Europäisches Patentamt
European Patent Office
Office européen des brevets

(11) EP 0 819 424 B2

(12) NOUVEAU FASCICULE DE BREVET EUROPEEN

(45) Date de publication et mention de la décision concernant l'opposition:
**13.08.2003 Bulletin 2003/33**

(45) Mention de la délivrance du brevet:
**22.03.2000 Bulletin 2000/12**

(21) Numéro de dépôt: **97401607.3**

(22) Date de dépôt: **04.07.1997**

(51) Int Cl.$^7$: **A61K 7/13**

# (54) Composition de teinture d'oxydation des fibres kératiniques et procédé de teinture mettant en oeuvre cette composition

Oxidationshaarfärbemittel und Färbeverfahren mit demselben Mittel

Oxidative hair dye composition and the dyeing process using such a composition

(84) Etats contractants désignés:
**DE ES FR GB IT**

(30) Priorité: **19.07.1996 FR 9609109**

(43) Date de publication de la demande:
**21.01.1998 Bulletin 1998/04**

(73) Titulaire: **L'OREAL**
**75008 Paris (FR)**

(72) Inventeur: **Audousset, Marie-Pascale**
**92600 Asnieres (FR)**

(74) Mandataire: **Goulard, Sophie et al**
**L'OREAL-DPI**
**6 rue Bertrand Sincholle**
**92585 Clichy Cedex (FR)**

(56) Documents cités:

**EP-A- 0 634 163**
**EP-A- 0 634 164**
**EP-A- 0 662 317**
**EP-A- 0 728 465**
**FR-A- 2 677 649**

EP 0 819 424 B2

## Description

L'invention a pour objet une composition pour la teinture d'oxydation des fibres kératiniques, en particulier des fibres kératiniques humaines telles que les cheveux, comprenant de la 2-n-propyl paraphénylènediamine, en association avec au moins un méta-aminophénol dans un rapport molaire méta-aminophénol / 2-n-propyl paraphénylènediamine inférieur ou égal à 10, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines; des ortho ou paraaminophénols, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet drfféremment sensibilisée (i.e. abîmée) entre sa pointe et sa racine.

Il a déjà été proposé, notamment dans la demande de EP 0 728 465, des compositions pour la teinture d'oxydation des fibres kératiniques renfermant certains coupleurs de type méta-aminophénols, en association avec au moins un précurseur de colorant d'oxydation de type paraphénylènediamine ou dérivés et un second coupleur de type 6-hydroxyindoline. Les colorations obtenues avec ces compositions ne sont toutefois pas entièrement satisfaisantes, notamment en ce qui concerne la tenue de ces colorations vis à vis des diverses agressions que peuvent subir les cheveux et en particulier les shampooings.

Il a également déjà été proposé, notamment dans la demande de brevet EP-A-0 634 163, des compositions pour la teinture d'oxydation des fibres kératiniques renfermant notamment de la 2-n-propyl paraphénylènediamine à titre de base d'oxydation en association avec une seconde base d'oxydation choisie parmi le 3-méthyl para-aminophénol, le 2-méthyl para-aminophénol et le 2-hydroxyméthyl para-aminophénol, et du 2-méthyl 5-N-(β-hydroxyéthyl) amino phénol à titre de coupleur, dans un rapport molaire 2-méthyl 5-N-(β-hydroxyéthyl)amino phénol / 2-n-propyl paraphénylènediamine nettement supérieur à 10. Ces compositions ne sont toutefois pas entièrement satisfaisantes, en particulier en ce qui concerne la puissance des colorations obtenues.

La présente invention vise à proposer de nouvelles compositions pour la coloration d'oxydation des fibres kératiniques et en particulier des fibres kératiniques humaines telles que les cheveux qui présentent de très bonnes propriétés tinctoriales.

Ainsi, la demanderesse vient maintenant de découvrir qu'il est possible d'obtenir de nouvelles teintures particulièrement résistantes, qui engendrent des colorations intenses, en associant

- à titre de seule base de la 2-n-propyl paraphénylènediamine et/ou l'un de ses sels d'addition avec un acide,
- à titre de seul coupleur un méta-aminophénol de formule (I) définie ci-après,

étant entendu que le rapport molaire méta-aminophénol de formule (I) / 2-n-propyl paraphénylènediamine est inférieur ou égal à 10.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :

- à titre de seule base d'oxydation, de la 2-n-propyl paraphénylènediamine et/ou au moins l'un de ses sels d'addition avec un acide,

- à titre de seul coupleur un coupleur choisi parmi les méta-aminophénols de formule (I) suivante, et leurs sels d'addition avec un acide :

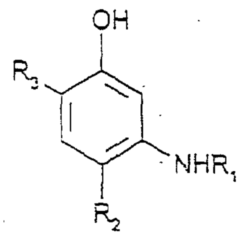

(I)

dans laquelle :

- $R_1$ représente un atome d'hydrogène, un radical alkyle en $C_1$-$C_4$, monohydroxyalkyle en $C_1$-$C_4$ ou polyhydroxyalkyle en $C_2$-$C_4$,
- $R_2$ représente un atome d'hydrogène, un radical alkyle en $C_1$-$C_4$, alcoxy en $C_1$-$C_4$ ou un atome d'halogène choisi parmi le chlore, le brome ou le fluor,
- $R_3$ représente un atome d'hydrogène, un radical alkyle en $C_1$-$C_4$, alcoxy en $C_1$-$C_4$, monohydroxyalkyle en $C_1$-$C_4$, polyhydroxyalkyle en $C_2$-$C_4$, monohydroxyalcoxy en $C_1$-$C_4$ ou polyhydroxyalcoxy en $C_2$-$C_4$,

étant entendu que :

- le rapport molaire méta-aminophénol de formule (I)/2-n-propyl paraphénylènediamine est inférieur ou égal à 10.

Les colorations obtenues avec les compositions ci-dessus présentent une bonne puissance tinctoriale et d'excellentes propriétés de résistance à la fois vis à vis des agents atmosphériques tels que la lumière et les intempéries et vis à vis de la transpiration et des différents traitements que peuvent subir les cheveux (shampooings, déformations permanentes). Ces propriétés sont particulièrement remarquables, notamment en ce qui concerne la résistance des colorations obtenues vis à vis des shampooings.

L'invention a également pour objet un procédé de teinture d'oxydation des fibres kératiniques mettant en oeuvre cette composition.

Selon une forme de réalisation préférée de l'invention, le rapport molaire méta-aminophénol de formule (I) /2-propyl paraphénylènediamine est inférieur ou égal à 5.

Les sels d'addition avec un acide utilisables dans le cadre des compositions tinctoriales de l'invention peuvent être notamment choisis parmi les chlorhydrates, les bromhydrates, les sulfates et les tartrates.

Parmi les méta-aminophénols de formule (I) ci-dessus, on peut plus particulièrement citer le méta-aminophénol, le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-(y-hydroxypropylamino) 2-méthyl phénol, et leurs sels d'addition avec un acide.

La 2-n-propyl paraphénylènediamine et/ou le ou les sels d'addition de ce composé avec un acide représentent de préférence de 0,0005 à 10 % en poids environ du poids total de la composition tinctoriale, et encore plus préférentiellement de 0,05 à 7 % en poids environ.

Le ou les méta-aminophénols formule (I) conformes à l'invention représentent de préférence de 0,0001 à 5 % en poids environ du poids total de la composition tinctoriale, et encore plus préférentiellement de 0,005 à 3 % en poids environ.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; le glycérol ; les glycols et éthers de glycols comme le 2-butoxyéthanol, le propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale telle que définie précédemment est généralement compris entre 5 et 12 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemples, les acides minéraux ou organiques comme

l'acide chlorhydrique, l'acide orthophosphorique, les acides carboxyliques comme l'acide tartrique, l'acide citrique, l'acide lactique, les acides sulfoniques.

Parmi les agents alcalinisants, on peut citer, à titre d'exemples, l'ammoniaque, les carbonates alcalins, les alcanolamines telles que les mono-, di- et triéthanolamines ainsi que leurs dérivés, les hydroxydes de sodium ou de potassium et les composés de formule (II) suivante :

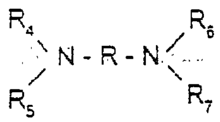

(II)

dans laquelle R est un reste propylène éventuellement substitué par un groupement hydroxyle ou un radical alkyle en $C_1$-$C_4$ ; $R_4$, $R_5$, $R_6$ et $R_7$, identiques ou différents, représentent un atome d'hydrogène, un radical alkyle en $C_1$-$C_4$ ou hydroxyalkyle en $C_1$-$C_4$.

La composition tinctoriale conforme à l'invention peut encore contenir, en plus des colorants définis ci-dessus, des colorants directs, notamment pour modifier les nuances ou les enrichir en reflets.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres à colorer la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de réalisation préférée de l'invention, le pH résultant du mélange de la composition tinctoriale et de la composition oxydante est compris entre 5 et 12 environ.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour lateinture, au moins un agent oxydant présent en une quantité suffisante pour développer une coloration. Le mélange obtenu est ensuite appliqué sur les fibres kératiniques et on laisse poser pendant 3 à 40 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 2 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser

une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-A-2 586 913 au nom de la demanderesse.

Les exemples qui suivent sont destinés à illustrer l'invention sans pour autant en limiter la portée.

## EXEMPLES

### EXEMPLES 1 et 2 COMPARATIFS

On a préparé les compositions tinctoriales suivantes (teneurs en grammes) :

| **EXEMPLE** | **1** | **2(*)** (comp) |
|---|---|---|
| Dichlorhydrate de 2-n-propyl paraphénylènediamine (Base d'oxydation) | 0,669 | - |
| Dichlorhydrate de 2,6-diméthyl paraphénylènediamine (Base d'oxydation) | - | 0,627 |
| 2-méthyl 5-amino phénol (coupleur) | 0,369 | 0.369 |
| Support de teinture commun (**) | (**) | (**) |
| Eau q.s.p. | 100 g | 100 g |

(*) : exemple ne faisant pas partie de l'invention

(**) support de teinture commun :
- Alcool oléique polyglycérolé à 2 moles de glycérol 4,0 g
- Alcool oléique polyglycérolé à 4 moles de glycérol, à 78 % de matières actives (M.A.) 5,69 g M.A.
- Acide oléique 3,0 g
- Amine oléique à 2 moles d'oxyde d'éthylène vendue sous la dénomination commerciale ETHOMEEN 012 par la société AKZO 7,0 g
- Laurylamino succinamate de diéthylaminopropyle, sel de sodium à 55 % de M.A. 3,0 g M.A.
- Alcool oléique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium en solution aqueuse, à 35 % de M.A. 0,455 g M.A.
- Acétate d'ammonium 0,8 g
- Antioxydant, séquestrant q.s.
- Parfum, conservateur q.s.
- Ammoniaque à 20 % de $NH_3$ 10,0 g

Il est important de noter que chaque composition tinctoriale contient la même quantité molaire de base d'oxydation, à savoir $3.10^{-3}$ mole.

Au moment de l'emploi, on a mélangé chaque composition tinctoriale avec une quantité égale d'une composition oxydante constituée par une solution d'eau oxygénée à 20 volumes (6 % en poids) et présentant un pH d'environ 3.

Chaque mélange obtenu présentait un pH d'environ 10.2 et a été appliqué pendant 30 minutes sur des mèches de cheveux gris permanentés. Les mèches de cheveux ont ensuite été rincées, lavées avec un shampooing standard puis séchées.

La couleur des mèches a ensuite été évaluée dans le système MUNSELL au moyen d'un colorimètre CM 2002 MINOLTA.

Selon la notation MUNSELL, une couleur est définie par l'expression H V/C dans laquelle les trois paramètres désignent respectivement la teinte ou Hue (H), l'intensité ou Value (V) et la pureté ou Chromaticité (C), la barre oblique de cette expression est simplement une convention et n'indique pas un ratio.

Les mèches de cheveux ainsi teintes ont ensuite été soumises à un test de résistance aux lavages (machine Ahiba-Texomat).

Pour ce faire, les mèches de cheveux ont été placées dans un panier que l'on a immergé dans une solution d'un shampooing standard. Le panier a été soumis à un mouvement de va-et-vient vertical de fréquence variable ainsi qu'à un mouvement de rotation qui reproduisent l'action d'un frottement manuel, ce qui engendre la formation de mousse.

Après 3 minutes d'épreuve, on a retiré les mèches que l'on a rincées puis séchées. Les mèches teintes ont été soumises à 6 épreuves de shampooing consécutives.

La couleur des mèches a été ensuite évaluée à nouveau dans le système MUNSELL au moyen d'un colorimètre CM 2002 MINOLTA.

La différence entre la couleur de la mèche avant les shampooings et la couleur de la mèche après les shampooings a été calculée en appliquant la formule de NICKERSON :

$$\Delta E = 0,4\ Co\Delta H + 6\Delta V + 3\ \Delta C.$$

telle que décrite par exemple dans "Couleur, Industrie et Technique" ; pages 14-17 ; vol. n° 5 ; 1978.

Dans cette formule, ΔE représente la différence de couleur entre deux mèches, ΔH, ΔV et ΔC représentent la variation en valeur absolue des paramètres H, V et C et Co représente la pureté de la mèche par rapport à laquelle on désire évaluer la différence de couleur.

Les résultats sont donnés dans le tableau ci-dessous :

| EXEMPLE | Couleur des cheveux avant les shampooings | Couleur des cheveux après les shampooings | Dégradation de la couleur | | | |
|---|---|---|---|---|---|---|
| | | | ΔH | ΔV | ΔC | **ΔE** |
| **1** | 3,4 RP 2,5/2,8 | 5 RP 3,3/2,2 | 1,6 | 0,8 | 0,6 | **8,4** |
| **2(*)** | 6 RP 2,6/2,4 | 1,1 YR 4,2/1,2 | 15,1 | 1,6 | 1,2 | **27,7** |

(*) : Exemple ne faisant pas partie de l'invention.

Ces résultats montrent que la composition 1 contenant l'association de la 2-n-propyl paraphénylènediamine et du 2-méthyl amino phénol conforme à l'invention conduit à une coloration résistant beaucoup mieux aux shampooings que la composition 2 contenant une base d'oxydation ne faisant pas partie de l'invention, à savoir la 2,6-diméthyl paraphénylènediamine et telle que décrite par exemple dans la demande de brevet européen EP-A-0 634 164.

**EXEMPLES 3 et 4 COMPARATIFS**

On a préparé les compositions tinctoriales suivantes (teneurs en moles) :

| **EXEMPLE** | **3** | **4(*)** |
|---|---|---|
| Dichlorhydrate de 2-n-propyl paraphénylènediamine (Base d'oxydation) | $4,79.10^{-4}$ | $4.48.10^{-5}$ |
| 2-méthyl 5-N-(β-hydroxyéthyl)amino phénol (Coupleur) | $4,79,10^{-3}$ | $4.79.10^{-3}$ |
| **Rapport molaire Coupleur / Base d'oxydation** | **10** | **107** |
| Support de teinture commun (**) | (**) | (**) |
| Eau q.s.p. | 100 g | 100 g |

(*) : exemple ne faisant pas partie de l'invention

(**) support de teinture commun :
Il est identique à celui des exemples 1 et 2 ci-dessus.

Au moment de l'emploi, on a mélangé chaque composition tinctoriale avec une quantité égale d'une composition oxydante constituée par une solution d'eau oxygénée à 20 volumes (6 % en poids) et présentant un pH d'environ 3.

Chaque mélange obtenu présentait un pH d'environ 10,2 et a été appliqué sur des mèches de cheveux gris naturels à 90 % de blancs selon le procédé de teinture décrits précédemment pour les exemples 1 et 2.

La couleur des mèches a été évaluée dans le système MUNSELL au moyen d'un colorimètre CM 2002 MINOLTA avant et après la teinture.

La différence ΔE entre la couleur de la mèche avant la teinture et la couleur de la mèche après la teinture a été calculée en appliquant la formule de NICKERSON et reflète la puissante de la coloration obtenue.

Les résultats sont donnés dans le tableau ci-dessous :

| EXEMPLE | Couleur des cheveux avant la teinture | Couleur des cheveux après la teinture | Montée de la coloration | | | |
|---|---|---|---|---|---|---|
| | | | ΔH | ΔV | ΔC | **ΔE** |
| **3** | 3,8 Y 5,5/1,2 | 9,6 RP 4,3/2,2 | 24.2 | 1,2 | 1,0 | **21,8** |
| **4(*)** | 3,8 Y 5,5/1,2 | 5,7 YR 5,4/1,7 | 8,1 | 0,1 | 0,5 | **6,0** |

(*) : Exemple ne faisant pas paie de l'invention.

Ces résultats montrent que la composition tinctoriale de l'exemple 3 conforme à l'invention, c'est dire contenant l'association de la 2-n-propyl paraphénylènediamine et du 2-méthyl 5-N-(β-hydroxyéthyl)amino phénol dans un rapport molaire coupleur / base égal à 10 confère aux cheveux une coloration beaucoup plus puissante que la composition de l'exemple 4 ne faisant pas partie de l'invention car contenant cette même association mais dans un rapport molaire égal à 107 et telle que décrite par exemple dans la demande de brevet européen EP-A-0 634 163.

## Revendications

**1.** Composition pour la teinture d'oxydation des fibres kératiniques et en particulier des fibres kératiniques humaines telles que les cheveux, **caractérisée par le fait qu'**elle comprend, dans un milieu approprié pour la teinture :

- à titre de seule base d'oxydation, de la 2-n-propyl paraphénylènediamine et/ou au moins l'un de ses sels d'addition avec un acide,
- à titre de seul coupleur un coupleur choisi parmi les méta-aminophénols de formule (I) suivante, et leurs sels d'addition avec un acide :

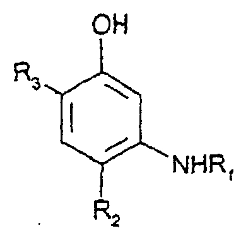

(I)

dans laquelle:

- $R_1$ représente un atome d'hydrogène, un radical alkyle en $C_1$-$C_4$, monohydroxyalkyle en $C_1$-$C_4$ ou polyhydroxyalkyle en $C_2$-$C_4$,
- $R_2$ représente un atome d'hydrogène, un radical alkyle en $C_1$-$C_4$, alcoxy en $C_1$-$C_4$ ou un atome d'halogène choisi parmi le chlore, le brome ou le fluor,
- $R_3$ représente un atome d'hydrogène, un radical alkyle en $C_1$-$C_4$, alcoxy en $C_1$-$C_4$, monohydroxyalkyle en $C_1$-$C_4$, polyhydroxyalkyle en $C_2$-$C_4$, monohydroxyalcoxy en $C_1$-$C_4$ ou polyhydroxyalcoxy en $C_2$-$C_4$,

étant entendu que :

- le rapport molaire méta-aminophénol de formule (I) / 2-n-propyl paraphénylènediamine est inférieur ou égal. à 10.

**2.** Composition selon la revendication 1, **caractérisée par le fait que** le rapport molaire méta-aminophénol de formule (I) / 2-n-propyl paraphénylènediamine est inférieur ou égal à 5.

**3.** Composition selon la revendication 1 ou 2, **caractérisée par le fait que** les méta-aminophénols de formule (I) sont choisie parmi le méta-aminophénol, le 5-amino 2-méthoxy phénol, le 5-amino 2-(β-hydroxyéthyloxy) phénol, le 5-amino 2-méthyl phénol, le 5-N-(β-hydroxyéthyl)amino 2-méthy) phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-(γ-hydroxypropylamino) 2-méthyl phénol, et leurs sels d'addition avec un acide.

**4.** Composition selon l'une quelconque des revendications précédentes, **caractérisée par le fait que** les sels d'addition avec un acide sont choisis parmi les chlorhydrates, les bromhydrates, les sulfates et les tartrates.

**5.** Composition selon l'uns quelconque des revendications précédentes, **caractérisée par le fait que** la 2-n-propyl paraphénylènediamine et/ou le ou les sels d'addition de ce composé avec un acide représentent de 0,0005 à 10 % en poids du poids total de la composition tinctoriale.

**6.** Composition selon la revendication 5, **caractérisée par le fait que** la 2-n-propyl paraphénylènediamine et/ou le ou les sels d'addition de ce composé avec un acide représentent de 0,05 à 7 % en poids du poids total de la composition tinctoriale.

**7.** Composition selon l'une quelconque des revendications précédentes, **caractérisée par le fait que** le ou les métaaminophénols de formule (I) représentent de 0,0001 à 5 % en poids du poids total de la composition tinctoriale.

**8.** Composition selon la revendication 7, **caractérisée par le fait que** le ou les méta-aminophénols de formule (I) représentent de 0,005 à 3 % en poids du poids total de la composition tinctoriale.

**9.** Composition selon l'une quelconque des revendications précédentes, **caractérisée par le fait que** ledit milieu approprié pour la teinture est constitué par de l'eau ou par un mélange d'eau et d'au moins un solvant organique choisi parmi les alcanols inférieurs en $C_1$-$C_4$, le glycérol, les glycols et éthers de glycols, les alcools aromatiques, les produits analogues et leurs mélanges.

**10.** Composition selon l'une quelconque des revendications précédentes, **caractérisée par le fait qu'**elle présente un pH compris entre 5 et 12.

**11.** Composition selon l'une quelconque des revendications précédentes, **caractérisée par le fait qu'**elle contient, en outre, des colorants directs.

**12.** Procédé de teinture des fibres kératiniques et en particulier des fibres kératiniques humaines telles que les cheveux **caractérisé par le fait que** l'on applique sur ces fibres une composition tinctoriale telle que définie à l'une quelconque des revendications 1 à 11 et que l'on révèle la couleur à pH acide, neutre ou alcalin à l'aide d'un agent oxydant qui est ajouté juste au moment de l'emploi à la composition tinctoriale ou qui est présent dans une composition oxydante appliqués simultanément ou séquentiellement de façon séparée. _

**13.** Procédé selon la revendication 12, **caractérisé par le fait que** l'agent oxydant présent dans la composition oxydante est choisi parmi le peroxyde d'hydrogène, le peroxyde d'urée, les bromates de métaux alcalins, les persels tels que les perborates et persulfates.

**14.** Dispositif à plusieurs compartiments ou "kit" de teinture à plusieurs compartiments dont un premier compartiment renferme une composition tinctoriale telle que définie à l'une quelconque des revendications 1 à 11 et un second compartiment renferme une composition oxydante comprenant un agent oxydant.

## Patentansprüche

**1.** Zusammensetzung zum oxidativen Färben von Keratinfasern und insbesondere von menschlichen Keratinfasern, wie dem Haar, **dadurch gekennzeichnet, dass** sie in einem zum Färben geeigneten Medium enthält:

- als einzige Oxidationsbase das 2-n-Propyl-p-phenylendiamin und/oder mindestens ein Additionssalz dieser Verbindung mit einer Säure, und

- als einzigen Kuppler einen Kuppler, der unter den m-Aminophenolen der folgenden Formel (I) oder den Additionssalzen dieser Verbindungen mit einer Säure ausgewählt ist:

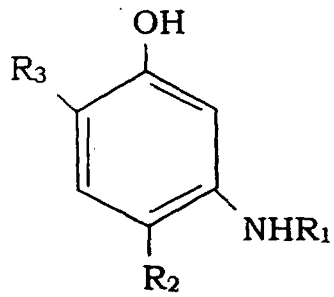

(I)

worin bedeuten:

- $R_1$ Wasserstoff, $C_{1-4}$-Alkyl, $C_{1-4}$-Monohydroxyalkyl oder $C_{2-4}$-Polyhydroxyalkyl,
- $R_2$ Wasserstoff, $C_{1-4}$-Alkyl, $C_{1-4}$-Alkoxy oder ein Halogenatom, das unter Chlor, Brom oder Fluor ausgewählt ist,
- $R_3$ Wasserstoff, $C_{1-4}$-Alkyl, $C_{1-4}$-Alkoxy, $C_{1-4}$-Monohydroxyalkyl, $C_{2-4}$-Polyhydroxyalkyl, $C_{1-4}$-Monohydroxyalkoxy oder $C_{2-4}$-Polyhydroxyalkoxy,

mit der Maßgabe, dass

- das Molverhältnis m-Aminophenol der Formel (I)/2-n-Propyl-pphenylendiamin höchstens 10 beträgt.

**2.** Zusammensetzung nach Anspruch 1, **dadurch gekennzeichnet, dass** das Molverhältnis m-Aminophenol der Formel (I)/2-n-Propylp-phenylendiamin höchstens 5 beträgt.

**3.** Zusammensetzung nach Anspruch 1 oder 2, **dadurch gekennzeichnet, dass** die m-Aminophenole der Formel (I) unter m-Aminophenol, 5-Amino-2-methoxyphenol, 5-Amino-2-(β-hydroxyethyloxy)-phenol, 5-Amino-2-methylphenol, 5-N-(β-Hydroxyethyl)amino-2-methyl-phenol, 5-N-(β-Hydroxyethyl)amino-4-methoxy-2-methylphenol, 5-Amino-4-methoxy-2-methyl-phenol, 5-Amino-4-chlor-2-methyl-phenol, 5-Amino-2,4-dimethoxy-phenol, 5-(γ-Hydroxypropylamino)-2-methyl-phenol und den Additionssalzen dieser Verbindungen mit einer Säure ausgewählt sind.

**4.** Zusammensetzung nach einem der vorhergehenden Ansprüche, **dadurch gekennzeichnet, dass** die Additionssalze mit einer Säure unter den Hydrochloriden, Hydrobromiden, Sulfaten und Tartraten ausgewählt sind.

**5.** Zusammensetzung nach einem der vorhergehenden Ansprüche, **dadurch gekennzeichnet, dass** das 2-n-Propyl-p-phenylendiamin und/oder das Additionssalz oder die Additionssalze dieser Verbindung mit einer Säure 0,0005 bis 10 Gew.-% des Gesamtgewichts der Farbmittelzusammensetzung ausmachen.

**6.** Zusammensetzung nach Anspruch 5, **dadurch gekennzeichnet, dass** das 2-n-Propyl-p-phenylendiamin und/oder das Additionssalz oder die Additionssalze dieser Verbindung mit einer Säure 0,05 bis 7 Gew.-% des Gesamtgewichts der Farbmittelzusammensetzung ausmachen.

**7.** Zusammensetzung nach einem der vorhergehenden Ansprüche, **dadurch gekennzeichnet, dass** das m-Aminophenol oder die m-Aminophenole der Formel (I) 0,0001 bis 5 Gew.-% des Gesamtgewichts der Farbmittelzusammensetzung ausmachen.

**8.** Zusammensetzung nach Anspruch 7, **dadurch gekennzeichnet, dass** das m-Aminophenol oder die m-Aminophenole der Formel (I) 0,005 bis 3 Gew.-% des Gesamtgewichts der Farbmittelzusammensetzung ausmachen.

**9.** Zusammensetzung nach einem der vorhergehenden Ansprüche, **dadurch gekennzeichnet, dass** das zum Färben geeignete Medium aus Wasser oder einem Gemisch von Wasser und mindestens einem organischen Lösungsmittel besteht, das unter den niederen $C_{1-4}$-Alkanolen, Glycerin, Glykolen und Glykolethern, aromatischen Alkoholen, analogen Produkten und deren Gemischen ausgewählt ist.

**10.** Zusammensetzung nach einem der vorhergehenden Ansprüche, **dadurch gekennzeichnet, dass** sie einen

pH-Wert im Bereich von 5 bis 12 aufweist.

**11.** Zusammensetzung nach einem der vorhergehenden Ansprüche, **dadurch gekennzeichnet, dass** sie ferner Direktfarbstoffe enthält.

**12.** Verfahren zum Färben von Keratinfasern und insbesondere menschlichen Keratinfasern, wie dem Haar, **dadurch gekennzeichnet, dass** auf die Fasern eine Farbmittelzusammensetzung nach einem der Ansprüche 1 bis 11 aufgebracht wird und die Farbe bei einem sauren, neutralen oder alkalischen pH-Wert mit einem Oxidationsmittel entwickelt wird, das unmittelbar bei der Anwendung zu der Farbmittelzusammensetzung gegeben wird oder das in einer oxidierenden Zusammensetzung vorliegt, die gleichzeitig oder getrennt davon anschließend aufgebracht wird.

**13.** Verfahren nach Anspruch 12, **dadurch gekennzeichnet, dass** das in der oxidierenden Zusammensetzung vorliegende Oxidationsmittel unter Wasserstoffperoxid, Harnstoffperoxid, Alkalimetallbromaten und Salzen von Persäuren, wie Perboraten und Persulfaten, ausgewählt ist.

**14.** Vorrichtung mit mehreren Abteilungen oder "Kit" zum Färben, wobei eine erste Abteilung die Farbmittelzusammensetzung nach einem der Ansprüche 1 bis 11 und eine zweite Abteilung eine oxidierende Zusammensetzung mit dem Oxidationsmittel enthält.

**Claims**

**1.** Composition for the oxidation dyeing of keratin fibres, and in particular of human keratin fibres such as the hair, **characterized in that** it comprises, in an appropriate medium for dyeing:

- as the only oxidation base, 2-n-propyl-para-phenylenediamine and/or at least one of the addition salts thereof with an acid,
- as the only coupler, a coupler chosen from the meta-aminophenols of formula (I) below, and the addition salts thereof with an acid:

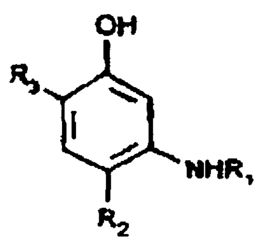

(I)

in which:

- $R_1$ represents a hydrogen atom or a $C_1$-$C_4$ alkyl, $C_1$-$C_4$ monohydroxyalkyl or $C_2$-$C_4$ polyhydroxyalkyl radical,
- $R_2$ represents a hydrogen atom, a $C_1$-$C_4$ alkyl or $C_1$-$C_4$ alkoxy radical or a halogen atom chosen from chlorine, bromine and fluorine,
- $R_3$ represents a hydrogen atom or a $C_1$-$C_4$ alkyl, $C_1$-$C_4$ alkoxy, $C_1$-$C_4$ monohydroxyalkyl, $C_2$-$C_4$ polyhydroxyalkyl, $C_1$-$C_4$ monohydroxyalkoxy or $C_2$-$C_4$ polyhydroxyalkoxy radical,

it being understood that:

- the meta-aminophenol of formula (I)/2-n-propyl-para-phenylenediamine molar ratio is less than or equal to 10.

**2.** Composition according to Claim 1, **characterized in that** the meta-aminophenol of formula (I)/2-n-propyl-para-phenylenediamine molar ratio is less than or equal to 5.

**3.** Composition according to Claim 1 or 2; **characterized in that** the meta-aminophenols of formula (I) are chosen

from meta-aminophenol, 5-amino-2-methoxyphenol, 5-amino-2-(β-hydroxyethyloxy)phenol, 5-amino-2-methylphenol, 5-N-(β-hydroxyethyl)amino-2-methylphenol, 5-N-(β-hydroxyethyl)amino-4-methoxy-2-methylphenol, 5-amino-4-methoxy-2-methylphenol, 5-amino-4-chloro-2-methylphenol, 5-amino-2,4-dimethoxyphenol and 5-(γ-hydroxypropylamino)-2-methylphenol, and the addition salts thereof with an acid.

**4.** Composition according to any one of the preceding claims, **characterized in that** the addition salts with an acid are chosen from the hydrochlorides, hydrobromides, sulphates and tartrates.

**5.** Composition according to any one of the preceding claims, **characterized in that** the 2-n-propyl-para-phenylenediamine and/or the addition salt or salts of this compound with an acid represent from 0.0005 to 10% by weight relative to the total weight of the dye composition.

**6.** Composition according to Claim 5, **characterized in that** the 2-n-propyl-para-phenylenediamine and/or the addition salt or salts of this compound with an acid represent from 0.05 to 7% by weight relative to the total weight of the dye composition.

**7.** Composition according to any one of the preceding claims, **characterized in that** the metaaminophenol(s) of formula (I) represent from 0.0001 to 5% by weight relative to the total weight of the dye composition.

**8.** Composition according to Claim 7, **characterized in that** the meta-aminophenol(s) of formula (I) represent from 0.005 to 3% by weight relative to the total weight of the dye composition.

**9.** Composition according to any one of the preceding claims, **characterized in that** the said appropriate medium for the dyeing consists of water or of a mixture of water and at least one organic solvent chosen from $C_1$-$C_4$ lower alkanols, glycerol, glycols and glycol ethers, aromatic alcohols, similar products and mixtures thereof.

**10.** Composition according to any one of the preceding claims, **characterized in that** it has a pH of between 5 and 12.

**11.** Composition according to any one of the preceding claims, **characterized in that** it also contains direct dyes.

**12.** Process for dyeing keratin fibres, and in particular human keratin fibres such as the hair, **characterized in that** a dye composition as defined in any one of Claims 1 to 11 is applied to these fibres and the colour is developed at acidic, neutral or alkaline pH using an oxidizing agent which is added only at the time of use to the dye composition or which is present in an oxidizing composition that is applied simultaneously or sequentially in a separate manner.

**13.** Process according to Claim 12, **characterized in that** the oxidizing agent present in the oxidizing composition is chosen from hydrogen peroxide, urea peroxide, alkali metal bromates and persalts such as perborates and persulphates.

**14.** Multi-compartment device for dyeing or multicompartment dyeing "kit", a first compartment of which contains a dye composition as defined in any one of Claims 1 to 11, and a second compartment of which contains an oxidizing composition comprising an oxidizing agent.